# EUROPÄISCHE PATENTSCHRIFT

(11) **EP 2 906 122 B1**
(45) Veröffentlichungstag und Bekanntmachung des Hinweises auf die Patenterteilung: **21.12.2016**
(21) Anmeldenummer: 13786632.3
(22) Anmeldetag: 08.10.2013
(51) Int. Cl.: A61B 6/10

(54) **STRAHLENSCHUTZANORDNUNG**
RADIATION PROTECTION ARRANGEMENT
ENSEMBLE DE PROTECTION CONTRE LES RADIATIONS

(30) Priorität: 09.10.2012 DE 102012218391
(43) Veröffentlichungstag der Anmeldung: 19.08.2015
(73) Patentinhaber: Mavig GmbH, 81829 München (DE)
(72) Erfinder: BUCHMEYER, Markus, 81829 München (DE)
(74) Vertreter: Vossius & Partner Patentanwälte Rechtsanwälte mbB
(86) Internationale Anmeldenummer: PCT/EP2013/070977
(87) Internationale Veröffentlichungsnummer: WO 2014/056940

(56) Entgegenhaltungen:
- WO-A1-01/84558
- DE-A1- 3 012 463
- DE-A1- 3 326 880
- DE-A1- 10 325 567
- DE-A1-102009 025 380
- DE-A1-102009 057 366

## Beschreibung

Die Erfindung betrifft eine Strahlenschutzanordnung insbesondere zur Anbringung an einer Trägerschiene, die an der Seite eines Behandlungstisches angebracht ist, als Unterkörperschutz. Diese Strahlenschutzanordnung ist insbesondere geeignet zur Verwendung in der interventionellen Radiologie und bei Operationen dient zum Schutz von beteiligtem Personal wie dem Arzt oder Assistenten gegen auftretende Strahlung, insbesondere Röntgenstrahlung.

Die DE 10 2009 025 380 A1 betrifft eine Strahlenschutzanordnung insbesondere zur Anbringung an einer Trägerschiene, die an der Seite eines Behandlungstisches angebracht ist. Die Strahlenschutzanordnung weist mindestens eine Lamelle aus einem Strahlenschutzmaterial und eine zugehörige Befestigungsvorrichtung auf. Die Lamelle ist an der Befestigungsvorrichtung schwenkbar befestigt. Die Befestigungsvorrichtung weist einen Halter auf, der auf die Trägerschiene aufsetzbar ist und mit einer Feststelleinrichtung ist der Halter an der Trägerschiene fixierbar.

Die WO 2004/107979 betrifft eine Strahlenschutzanordnung, die seitlich an einem medizinischen Untersuchungs- oder Behandlungstisch als Unterkörperschutz befestigbar ist. In einer Ausführungsform weist diese Strahlenschutzanordnung mehrere nebeneinander angeordnete Lamellen auf, die an einem Ende an einem gemeinsamen Trägerelement befestigt sind. Zusätzlich ist ein Oberteil vorgesehen, das an der Trägerschiene an dem Behandlungstisch befestigbar ist. Die Lamellen können bei dieser bekannten Strahlenschutzanordnung aus einer Bleigummimatte bestehen, die beispielsweise in PVC eingeschichtete Bleifolien aufweisen. Die Bleigummimatten werden in Umhüllungen eingesetzt, die aus einem einfach zu reinigenden und zu sterilisierenden Stoff bestehen. Die Lamellen sind in ihrer Länge veränderbar, in dem ein unteres Ende nach oben umgeschlagen wird und dieses Ende in dieser umgeschlagenen Position fixiert wird, beispielsweise mittels Druckknöpfen, Klettverschluss oder durch die Verwendung von Gurten.

Die DE 1 516 420 beschreibt eine Strahlenschutzvorrichtung mit mehreren einzelnen Bleigummilappen, die schwenkbar gelagert sind. Jeder Bleigummilappen ist um eine zu ihm senkrecht stehende, oberhalb seines Schwerpunktes befindliche Achse schwenkbar gelagert. In einer Ausführungsform ist ein kammartiger Träger vorhanden, bei dem an den Fortsätzen Zapfen vorhanden sind, an denen die einzelnen Bleigummilappen schwenkbar befestigt sind.

Die DE 1 466 848 beschreibt eine Strahlenschutzvorrichtung, bei der Bleigummilappen über Drehachsen an der Seitenkante eines Leuchtschirmträgers angebracht sind.

In der DE 27 49 826 A1 wird eine Röntgenstrahl-Abschirmeinrichtung beschrieben, bei denen Bleigummilappen vorhanden sind, die mit Klemmen um Achsen schwenkbar angebracht sind. In einer Ausführungsform weist eine Laufschiene ein T-förmiges Innenprofil auf, in das Halter einsetzbar sind.

Die DE 30 12 463 C2 zeigt eine Strahlenschutzvorrichtung, bei der Bleigummilappen schwenkbar an einem Träger aufgehängt sind. Bei einer Ausführungsform weist ein Tragelement kammartige Fortsätze auf, an denen die Bleigummilappen gelenkig gelagert sind.

Die DE 297 04 613 U1 beschreibt eine Strahlenschutzvorrichtung, bei der einzelne Bleigummilappen verschwenkbar an einer Schiene angeordnet werden können. In einer Ausführungsform ist für jeden Bleigummilappen ein Träger vorhanden, der in einer Führungsschiene verstellbar gelagert ist. Die Bleigummilappen sind an einem Träger um eine Querachse schwenkbar gelagert. Weiterhin sind Bremsmittel vorgesehen, mit denen verhindert wird, dass die Träger innerhalb der Schiene ungewollt verschoben werden. Alternativ kann hierzu eine Raste oder eine Sperre eingesetzt werden. In einer weiteren Ausführungsform weist jeder Bleigummilappen eine Öse mit einem Durchgangsloch auf, an der der Bleigummilappen von einer Schwenkachse gehalten wird.

Der Erfindung liegt die Aufgabe zugrunde, eine Strahlenschutzanordnung bereitzustellen, die einfach an einem Behandlungstisch anzubringen und einfach handbar ist. Desweiteren ist es eine Aufgabe der Erfindung einen einfachen und sicheren Verschluss für die Verriegelung der Strahlenschutzanordnung an dem Behandlungstisch, sowie ein komfortables Tragesystem der Strahlenschutzanordnung bereitzustellen.

Diese Aufgabe wird mit einer Strahlenschutzanordnung gemäß den Patentansprüchen gelöst.

Die erfindungsgemäße Strahlenschutzanordnung eignet sich insbesondere zur Anbringung an einer Trägerschiene, die an der Seite eines Behandlungstisches angebracht ist.

Die Erfindung geht von dem Grundgedanken aus, eine Strahlenschutzanordnung mit einem zugehörigen Halter durch einen geeigneten Mechanismus an einer gewünschten Position an der Trägerschiene des Behandlungstisches zu verriegeln und somit ein Abrutschen der Strahlenschutzanordnung zu verhindern, wobei der Mechanismus durch ein oder mehrere Haltegriffe betätigt wird.

Der/Die Haltegriff(e) werden von dem Fachpersonal zum komfortablen Tragen der Strahlenschutzanordnung verwendet und durch eine einfache Schwenkbewegung des/der Haltegriff(e) kann sobald die Strahlenschutzanordnung auf die Trägerschiene aufgesetzt wird, der Mechanismus betätigt und somit die Strahlenschutzanordnung an der Trägerschiene verriegelt werden.

Dabei hat die Erfindung den Vorteil, dass die feste Verriegelung der Strahlenschutzanordnung an die Trägerschiene schnell und sicher ausgeführt werden kann. Die Strahlenschutzanordnung muss für das Verriegeln nicht abgesetzt werden. Bei der Verriegelung befindet sich immer mindestens eine Hand des Fachpersonals an der Strahlenschutzanordnung. Somit kann ein unbeabsichtigtes Herunterfallen der Strahlenschutzanordnung verhindert werden.

Die Erfindung hat auch den Vorteil, dass bei der Verriegelung der Strahlenschutzanordnung an der Trägerschiene das Verletzungsrisiko des Fachpersonals, durch Verletzung während der Verriegelung, z.B. Quetschung der Finger des Fachpersonals, verhindert werden kann und auch das Tragen der Strahlenschutzanordnung von dem Behandlungstisch weg oder zu dem Behandlungstisch hin komfortabel gestaltet wird.

Der/Die Haltegriff(e) sind vorzugsweise an der Seite der Strahlenschutzanordnung angebracht, so dass bei dieser Verriegelung auf die Verwendung von Drehgriffen oder anderen Montageeinrichtungen an der dem Fachpersonal zugewandten Seite der Strahlenschutzanordnung verzichtet werden kann. Dies führt dazu, dass sich das Fachpersonal an diesen Drehgriffen oder ähnlichem nicht stoßen.

Der/Die Haltegriff(e) sind vorzugsweise beim Tragen der Strahlenschutzanordnung in einer Position in einem Winkel von 10°-70°, vorzugsweise 30°, hochgeklappt in Relation zur Längsrichtung des Halters, so dass sich für das Fachpersonal möglichst wenig Belastung am Handgelenk einstellt und ein optimaler Tragkomfort gewährleistet werden kann.

Die Erfindung betrifft eine Strahlenschutzanordnung insbesondere zur Anbringung an mindestens einer Trägerschiene, die vorzugsweise an einer oder mehreren Seiten eines Behandlungstisches angebracht ist, mit einer Befestigungsvorrichtung an der mindestens eine Lamelle, die ein Material zum Schutz vor Strahlung, vorzugsweise Röntgenstrahlung aufweist, vorzugsweise schwenkbar befestigt ist, wobei die Befestigungsvorrichtung einen Halter, der auf die Trägerschiene aufsetzbar ist, aufweist, wobei der Halter aufweist: mindestens einen schwenkbar gelagerten Haltegriff, wobei der Haltegriff ein freies Ende und ein Ende, an dem der Haltegriff schwenkbar gelagert ist, aufweist, einen Schließmechanismus geeignet zum Verriegeln des Halters an der Trägerschiene, wobei der Schließmechanismus durch eine Schwenkbewegung des mindestens einen an dem Halter schwenkbar gelagerten Haltegriffs betätigbar ist.

Die eine oder mehrere Seiten des Behandlungstisches umfassen dabei sowohl die Längs- als auch die Schmalseite des Behandlungstisches. Auch kann eine oder mehrere Lamellen fest an der Befestigungsvorrichtung angeordnet oder mindestens eine schwenkbar oder mindestens eine kardanisch an der Befestigungsvorrichtung aufgehängt sein. Auch ist eine beliebige Kombination der Anordnungs- bzw. Aufhängungsmöglichkeiten der einen oder mehreren Lamellen möglich.

In einer weiteren Ausführungsform steht der mindestens eine Haltegriff in direkter Wirkverbindung mit dem Schließmechanismus. Eine direkte Wirkverbindung bedeutet in diesem Zusammenhang, dass der Haltegriff direkt an den Schließmechanismus angrenzt und durch sein Wirken, beispielsweise eine Bewegung des Haltegriffes, der Schließmechanismus ausgelöst wird.

In einer weiteren Ausführungsform weist der Schließmechanismus mindestens einen Schließriegel auf, der geeignet ist den Halter der Strahlenschutzanordnung an der Trägerschiene zu verriegeln. Der Schließriegel kann eine rechteckige Grundfläche oder die Grundfläche eines Parallelogramms mit einer bestimmten Höhe oder eines Zylinders, vorzugsweise eines Kreiszylinders aufweisen.

In einer weiteren Ausführungsform ist der Haltegriff geeignet in eine erste Stellung geschwenkt zu werden. In der ersten Stellung ist der Halter an der Trägerschiene durch den Schließriegel nicht verriegelt.

In einer weiteren Ausführungsform ist der Haltegriff geeignet in eine zweite Stellung geschwenkt zu werden. In der zweiten Stellung verriegelt der mindestens eine Schließriegel den Halter an der Trägerschiene.

In einer weiteren Ausführungsform weist der Halter ein Längsprofil und eine Aufnahme auf, wobei das Längsprofil eine Auflagefläche aufweist, und wenn die Strahlenschutzanordnung auf die Trägerschiene aufgesteckt ist, die Auflagefläche des Längsprofils auf der Oberseite der Trägerschiene teilweise aufliegt und wobei vorzugsweise der mindestens eine Schließriegel sich in der Aufnahme befindet, die an der Auflagefläche des Längsprofils derart angeordnet ist, dass sich der mindestens eine Schließriegel in einem bestimmtem Abstand A zur Auflagefläche befindet.

In einer weiteren Ausführungsform ist der Abstand A derart ausgestaltet, dass wenn die Strahlenschutzanordnung auf die Trägerschiene aufgesteckt ist, der Schließriegel in einem verriegelten Zustand sich teilweise entlang der Unterseite der Trägerschiene erstreckt. Der Abstand A kann dabei identisch zur Dicke A' der Trägerschiene sein oder größer als die Dicke A' der Trägerschiene, vorzugsweise 0,05-3,0 mm größer, weiter bevorzugt 0,5-1,5 mm größer.

In einer weiteren Ausführungsform ist der Abstand A derart ausgestaltet, dass wenn die Strahlenschutzanordnung auf die Trägerschiene aufgesteckt ist, der Schließriegel im verriegelten Zustand an die Unterseite der Trägerschiene gedrückt ist, vorzugsweise in einem Winkel von 30° - 60°, vorzugsweise 45° zur Normalen der Unterseite der Trägerschiene. Auch kann der Schließriegel derart ausgestaltet sein, dass der Schließriegel an die dem Schließriegel am Nächsten liegende Kante der Trägerschiene in einem Winkel von 30° - 60°, vorzugsweise 45° zur Normalen der Unterseite gedrückt wird.

In einer weiteren Ausführungsform weist das Längsprofil eine kurze und eine lange Seite auf und der Haltegriff ist geeignet parallel zur langen Seite des Längsprofils des Halters zwischen der ersten und der zweiten Stellung hin und her geschwenkt zu werden.

In einer weiteren Ausführungsform weist das Längsprofil an einer Seite mindestens eine, vorzugsweise V-förmige, Aussparung auf. Auch kann das zulaufende Ende einer V-förmigen Aussparung abgerundet sein. Durch solch eine Abrundung des zulaufenden Ende der Aussparung kann insbesondere eine variable Positionierung der Strahlenschutzanordnung an der Trägerschiene gewährleistet sein.

In einer weiteren Ausführungsform ist der Haltegriff in der ersten Stellung in einem Winkel von 10° bis 70°, vorzugsweise 30°, zur Längsrichtung des Längsprofils des Halters bezogen auf die Oberseite des Längsprofils angeordnet.

In einer weiteren Ausführungsform ist der Haltegriff in der zweiten Stellung in einer Position in einem Winkel von etwa -90° zur Längsrichtung des Längsprofils des Halters bezogen auf die Oberseite des Längsprofils angeordnet ist.

In einer weiteren Ausführungsform ist der Haltegriff an der kurzen Seite des Längsprofils angebracht.

In einer weiteren Ausführungsform weist der Halter eine Lagerung auf, in der der Haltegriff schwenkbar gelagert ist.

In einer weiteren Ausführungsform weist der Haltegriff mindestens eine Rastaussparung auf, die geeignet ist den Haltegriff in der ersten und / oder der zweiten Stellung bis zu einer bestimmten Schwenkkraft zu halten.

In einer weiteren Ausführungsform weist der Haltegriff eine Abrutschsicherung auf, die geeignet ist ein Abrutschen einer Hand von dem Haltegriff in der zweiten Stellung zu verhindern, wobei die Abrutschsicherung vorzugsweise als Vorsprung an dem freien Ende des Haltegriffs ausgebildet ist.

In einer weiteren Ausführungsform weist der Schließmechanismus eine verschiebbare Stange auf.

Die Erfindung betrifft auch eine Strahlenschutzanordnung insbesondere zur Anbringung an mindestens einer Trägerschiene, die vorzugsweise an einer oder mehreren Seiten eines Behandlungstisches angebracht ist, mit einem Halter, der auf die Trägerschiene aufsetzbar ist und mindestens einer Aufsetzlamelle, die an unterschiedlichen Positionen auf dem Halter anbringbar ist, wobei vorzugsweise die Strahlenschutzanordnung mit einer der vorstehenden Strahlenschutzanordnungen kombinierbar ist.

In einer Ausführungsform weist der Halter mindestens eine Bohrung und die mindestens eine Aufsetzlamelle mindestens einen Zapfen auf. Dabei ist der mindestens eine Zapfen in die mindestens eine Bohrung einsteckbar.

Gemäß einer weiteren Ausführungsform weist die mindestens eine Aufsetzlamelle mindestens eine vorzugsweise U-förmige Halteeinrichtung auf. Die mindestens eine U-förmige Halteeinrichtung kann derart ausgestaltet sein, dass sie auf den Halter formschlüssig und/oder kraftschlüssig aufsetzbar ist. Die mindestens eine vorzugsweise U-förmige Halteeinrichtung kann dabei vorzugsweise aus einem Kunststoff hergestellt sein, besonders bevorzugt in der Form von Kunstoffclips. Die mindestens eine Aufsetzlamelle mit der vorzugsweise U-förmigen Halteeinrichtung kann derart ausgestaltet sein, dass sie auf dem Längsprofil der Strahlenschutzanordnung vorzugsweise gleitend verschiebbar ist. Durch die Verwendung der U-förmigen Halteeinrichtung, sowie der gleitenden Verschiebbarkeit ist eine variable Anbringung möglich und die Handhabung erleichtert. Außerdem ist das Risiko von etwaigen Lackbeschädigungen des Längsprofils der Strahlenschutzanordnung minimiert.

Die Aufsetzlamelle mit der mindestens einen U-förmigen Halteeinrichtung ist sowohl auf Halter mit Bohrungen als auch ohne Bohrungen aufsetzbar.

Der Begriff "Behandlungstisch" umfasst auch alle Arten von Patientenlagerungstischen, Untersuchungstischen oder Operationstischen.

Gemäß einer weiteren Ausführungsform kann die Strahlenschutzanordnung mindestens eine Querlamelle aufweisen, die ein Material zum Schutz vor Strahlung, vorzugsweise Röntgenstrahlung aufweist. Dabei kann das Material zum Schutz vor Strahlung, vorzugsweise Röntgenstrahlung, das gleiche Material sein, welches auch die mindestens eine Lamelle, die vorzugsweise schwenkbar befestigt ist, aufweist. Die mindestens eine Querlamelle kann an der Befestigungsvorrichtung angebracht sein. Insbesondere kann die mindestens eine Querlamelle an der dem Behandlungstisch abgewandten Wand des Längsprofils innen- und/oder außenseitig angebracht sein. Dabei ist die Querlamelle vorzugsweise so angebracht, dass sie sich über die lange Seite des Längsprofils mindestens teilweise erstreckt. Insbesondere kann die Querlamelle derart an dem Längsprofil angebracht sein, dass sie bei etwaigen Schwenkbewegungen der mindestens einen Lamelle die Befestigungsvorrichtung und den Teil der mindestens eine Lamelle, der an der Befestigungsvorrichtung aufgehängt ist, vollständig bedeckt. Des Weiteren kann die so ausgebildete mindestens eine Querlamelle zusätzlichen Schutz bei Schwenkbewegungen des/der Haltegriffe(s) gewährleisten. Die Querlamelle kann an dem Längsprofil verschraubt, genietet, oder verklebt sein. Auch kann die Querlamelle an der gleichen Befestigungsvorrichtung wie die mindestens eine Lamelle angebracht sein. So kann sowohl die Lamelle als auch die Querlamelle eine Öse aufweisen, und die Querlamelle und die mindestens eine Lamelle sind an derselben Befestigungsvorrichtung angebracht. Dabei kann die Befestigungsvorrichtung als Schraube ausgebildet sein, auf die die Ösen aufgesteckt sind und die Lamelle und die Querlamelle werden dementsprechend durch eine Mutter gesichert. Im Fall von mehreren Lamellen kann die Querlamelle an allen Befestigungsvorrichtungen der mehreren Lamellen angebracht sein oder auch nur einen Teil, beispielsweise im Fall von fünf Lamellen an den beiden äußeren Befestigungsvorrichtungen sowie der mittleren. Des Weiteren kann die Querlamelle an der dem Behandlungstisch abgewandten Seite des Trägers befestigt sein. Auch können im Fall von mehr als zwei Querlamellen, diese Querlamellen überlappend angebracht sein. Dabei kann die Querlamelle derart an den Träger befestigt sein, dass sie in Richtung des Behandlungstisches die Befestigungsvorrichtung der mindestens eine Lamelle sowie den Teil der mindestens einen Lamelle, der an der Befestigungsvorrichtung aufgehängt ist, vollständig bedeckt. Insbesondere kann bei der Verwendung von Aufsetzlamellen - die derart angebracht sind und vorzugsweise solche Abmessungen aufweisen, dass wenn die unteren Endabschnitte der Aufsetzlamellen überlappend mit den oberen Endabschnitten der Lamellen zueinander ausgerichtet sind, die Endabschnitte der Lamellen nicht abgedeckt sind - sichergestellt werden, dass durch diesen Bereich keine Strahlung dringt. Auch kann durch die Verwendung der mindestens einen Querlamelle gegebenenfalls auf eine Aufsetzlamelle verzichtet werden.

Die Erfindung wird nachstehend anhand von Beispielen und der Zeichnung näher erläutert. Es zeigen:
Figur 1 eine perspektivische Ansicht einer bevorzugten Ausführungsform einer Strahlenschutzanordnung gemäß der vorliegenden Erfindung,
Figur 2 eine vergrößerte Teilansicht der perspektivischen Ansicht von Figur 1,
Figur 3 eine weitere perspektivische Ansicht der ersten bevorzugten Ausführungsform einer Strahlenschutzanordnung gemäß der vorliegenden Erfindung,
Figur 4 eine Vorderansicht der ersten bevorzugten Ausführungsform einer Strahlenschutzanordnung gemäß der vorliegenden Erfindung,
Figur 5 eine perspektivische Ansicht eines Sets von zwei Strahlenschutzanordnungen im verriegelten Zustand an einer Trägerschiene an der Seite und am Kopfbereich eines Behandlungstisches (nicht dargestellt) gemäß einer zweiten bevorzugten Ausführungsform der vorliegenden Erfindung,
Figur 6a und 6b eine perspektivische Ansicht einer Strahlenschutzanordnung gemäß einer dritten bevorzugten Ausuhrungsform der vorliegenden Erfindung,
Figur 7a eine perspektivische Ansicht einer Aufsetzlamelle gemäß einer bevorzugten Ausführungsform gemäß der vorliegenden Erfindung und
Figur 7b eine perspektivische Ansicht einer Aufsetzlamelle gemäß einer weiteren Ausführungsform der vorliegenden Erfindung.

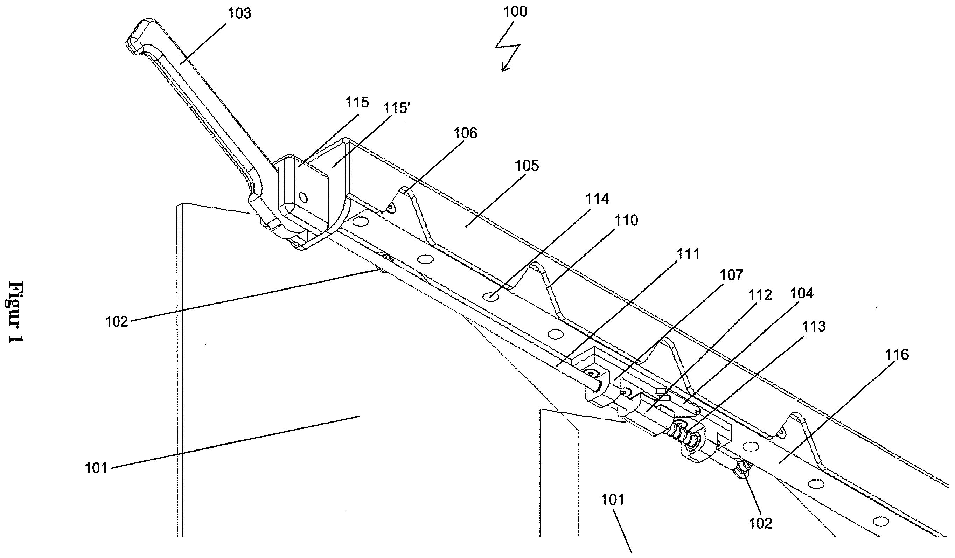

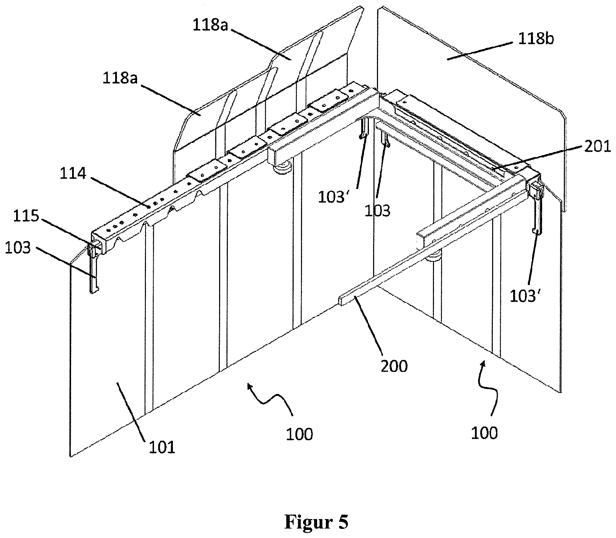

Figur 1 zeigt eine Ausführungsform einer Strahlenschutzanordnung 100 gemäß der vorliegenden Erfindung zur Anbringung an einen Behandlungstisch. Bei dem in Figur 1 gezeigten Beispiel sind zwei Lamellen 101 jeweils an einer entsprechenden Befestigungsvorrichtung 102 angeordnet. Die Strahlenschutzanordnung 100 weist ferner einen Halter auf, wobei der Halter wiederum ein Längsprofil 105 aufweist. Das Längsprofil 105 weist eine kurze und eine lange Seite auf. An dem Längsprofil 105 ist an der kurzen Seite seitlich ein Haltegriff 103 in einer Halterung 115 um eine Schwenkachse 115b schwenkbar gelagert. Gemäß der vorliegenden Ausführungsform ist die Halterung 115 mit dem Längsprofil 105 durch ein entsprechendes Verbindungsstück 115' verbunden. Das Längsprofil 105 weist ferner eine Auflagefläche 106 auf, wobei die Strahlenschutzanordnung 100, wenn diese auf eine Trägerschiene 200 (siehe Figur 5) aufgesteckt ist, teilweise mit der Auflagefläche 106 des Längsprofils 105 auf der Oberseite der Trägerschiene 200 aufliegt. Des Weiteren hat das Längsprofil 105 an der dem Behandlungstisch zugewandten Seite eine oder mehrere V-förmige Aussparungen 110, in die sich im aufgesteckten Zustand entsprechende Zapfen 201 (siehe Figur 5) der Trägerschiene 200 erstrecken. Somit kann verhindert werden, dass sich die Strahlenschutzanordnung 100 in seitlicher Richtung entlang der Trägerschiene 200 bzw. des Behandlungstisches ungewollt verschiebt. Des Weiteren weist der Halter einen Träger 116 auf, in dem sich in regelmäßigen Abständen Bohrungen in vertikaler Richtung befinden. Bohrungen mit gleichem Durchmesser sind deckungsgleich auch in dem Längsprofil 105 vorhanden. Durch diese Bohrungen können in dem aufgesteckten Zustand der Strahlenschutzanordnung 100 zusätzlich sich nach oben erstreckende Lamellen 118 (siehe Figur 5) aufgesetzt werden. Dies gewährleistet einen Schutz vor der Strahlung im Bereich des Oberkörpers des Fachpersonals, siehe Figur 5.

Figur 1 zeigt die Strahlenschutzanordnung 100 im unverriegelten d.h. geöffneten Zustand, bereit zum Aufstecken auf die Trägerschiene 200. In dem geöffneten Zustand ragt der Schließriegel 104 nicht oder nur in sehr geringem Maße über den Träger 116 in Richtung Trägerschiene 200 heraus. So kann die Strahlenschutzanordnung 100 derart auf die Trägerschiene 200 aufgesteckt werden, dass sich die Trägerschiene 200 zwischen dem Träger 116 und der dem Behandlungstisch zugewandten Innenseite des Längsprofils 105 befindet. Der Haltegriff 103 ist in dem unverriegelten Zustand hochgeklappt und befindet sich in einem Winkel in Relation zur langen Seite des Längsprofils des Halters in Richtung in einer ersten Stellung, einer offenen Position. Dieser Winkel beträgt vorzugsweise 10° bis 70°, besonders bevorzugt 30°. Daher stellt sich für das Fachpersonal möglichst wenig Belastung am Handgelenk beim Tragen der Strahlenschutzanordnung 100 ein und ein optimaler Tragkomfort kann gewährleistet werden.

Wie in Figur 1 gezeigt, befindet sich der Haltegriff 103 in Wirkverbindung mit einer Verbindungsstange 111, die sich in einer Aufnahme 107 erstreckt. Der Haltegriff 103 in der Halterung 115 nach unten in eine zweite Stellung um die Schwenkachse 115b geschwenkt. Die Stirnfläche des Haltegriffs 103 ist an der Seite, an der der Haltegriff 103 mit der Schwenkachse 115b an der Halterung 115' verbunden ist, als Exzenter 115a ausgebildet. Der Exzenter 115a drückt die Verbindungsstange 111 in Längsrichtung des Längsprofils nach innen gegen eine Feder 113. Die Feder sorgt dafür, dass im unverriegelten Zustand der Haltegriff 103 in seiner ersten Stellung verbleibt und nicht unbeabsichtigt einschwenkt. An der Verbindungsstange 111 ist außerdem ein Schieber 112 befestigt, der in Verbindung mit einem Schließriegel 104 steht. Dabei hat der Schließriegel 104 die Grundfläche eines Parallelogramms und weist eine bestimmte Höhe auf, wobei die Grundfläche des Schließriegels 104 dabei eine lange und eine kurze Seite besitzt. Die lange Seite ist in einem Winkel von 45° zum Träger 116 verschoben. Der Schließriegel 104 befindet sich durch eine Nut- und Federverbindung gleitend in der Aufnahme. Die Höhe des Schließriegels 104 ist so bemessen, dass der Schließriegel nicht bündig mit der Oberfläche der Aufnahme 107 abschließt. Dadurch kann der Schieber 112, der eine dem Schließriegel 104 entsprechende Aussparung, ebenfalls in einem Winkel von 45°, aufweist, eine Wirkverbindung mit dem Schließriegel eingehen. Die Wirkverbindung ist derart ausgestaltet, dass bei Bewegung der Verbindungsstange 111 der Schieber 112 den Schließriegel 104 in der Nut- und Federverbindung in Bezug auf das Längsprofil nach außen drückt. Die Bewegung der Verbindungsstange 111 wird dabei durch das Schwenken des als Exzenter 115a ausgebildeten Ende des Haltegriffs 103 bewirkt. Somit entsteht ein Schließmechanismus bei dem das Schwenken des Haltegriffs 103 zu einer Bewegung des Schließriegels 104 und schließlich zu einer Verriegelung der Strahlenschutzanordnung 100 an einer Trägerschiene 200 führt. Der Träger 116 und die Aufnahme 107 sind dabei so dimensioniert, dass sobald die Strahlenschutzanordnung 100 auf die Trägerschiene 200 aufgesteckt ist, sich der Schließriegel 104 im verriegelten Zustand teilweise oder ganz auf der Unterseite der Trägerschiene 200 erstreckt. Der Abstand des Schließriegels 104 zur Unterseite der Trägerschiene 200 beträgt dabei vorzugsweise 0 bis 1 mm, weiter bevorzugt 0,5 mm. Die Strahlenschutzanordnung 100 wird mit der Trägerschiene 200 verriegelt, so dass die Strahlenschutzanordnung 100 sich nicht unabsichtlich von der Trägerschiene 200 lösen kann.

Der Haltegriff 103 weist ferner eine Abrutschsicherung 109 auf, die sich an seinem freien Ende, d.h. gegenüberliegend zu dem Ende an dem der Haltegriff in der Halterung 115 schwenkbar gelagert ist, befindet. Ferner weist der Haltegriff 103 Rastaussparungen auf, wobei in Figur 1 nur eine Rastaussparung 108 gezeigt ist. Befindet sich der Haltegriff 103 in einer Position in der eine der Rastaussparung in Kontakt mit einer Kante der Halterung 115 ist, so ist der Haltegriff in dieser Position derart fixiert, so dass sich diese Position nur durch Überwindung eines gewissen Widerstandes, d.h. durch Überwindung einer bestimmten Schwenkkraft lösen lässt.

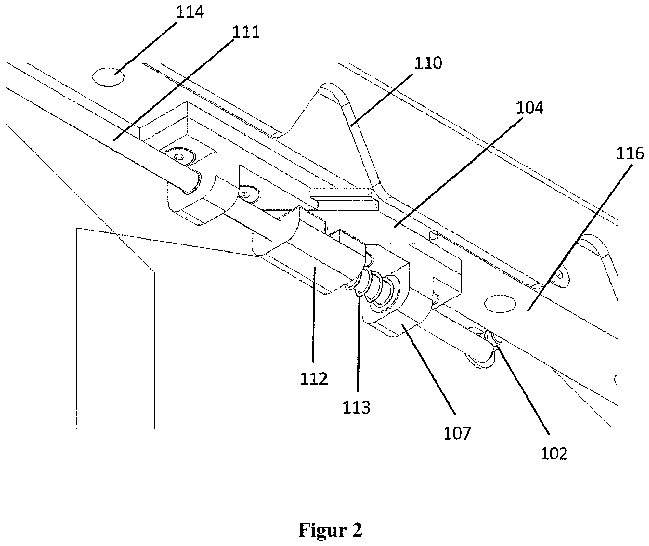

Figur 2 zeigt eine perspektivische Teilansicht eines Ausschnitts der Strahlenschutzanordnung 100 der ersten bevorzugten Ausführungsform gemäß der vorliegenden Erfindung. Im Einzelnen ist die Verbindungsstange 111, der an der Verbindungsstange 111 angebrachte Schieber 112, der mit dem Schieber 112 in Verbindung stehende Schließriegel 104 , der durch einen Nut- und Federmechanismus an der Aufnahme 107 angebracht ist, und der Träger 116 zu sehen. Gezeigt ist auch die Rückstellfeder 113, die dazu geeignet ist, durch geeigneten Druck auf den Schieber 112 den mit der Verbindungsstange 111 in Wirkverbindung stehenden Haltegriff 103 in der offenen Position, d.h. der ersten Stellung, zu halten.

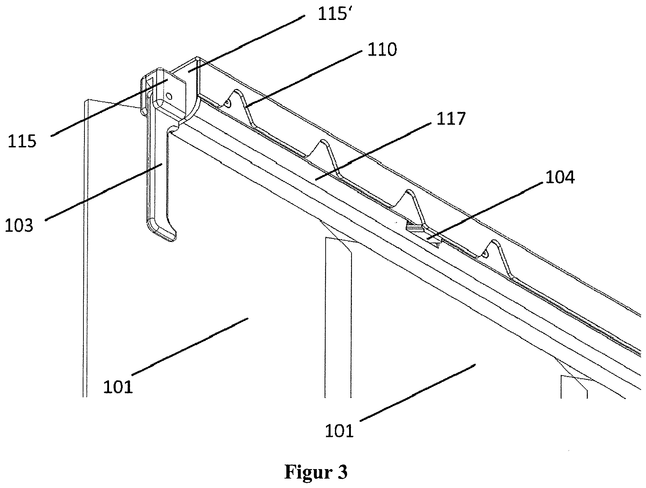

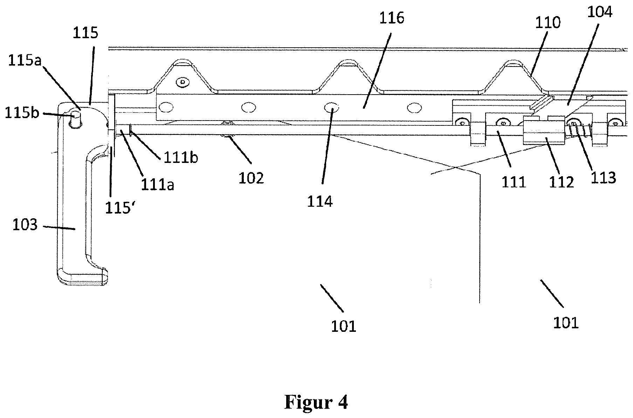

Figur 3 und 4 zeigen perspektivische Ansichten der ersten bevorzugten Ausführungsform einer Strahlenschutzanordnung gemäß der vorliegenden Erfindung. Im Unterschied zu Figur 1 und Figur 2 befindet sich eine Schutzabdeckung 117 über der Verbindungsstange 111, der Feder 113, der Aufnahme 107, dem Schieber 112, sowie teilweise über dem Schließriegel 104. Es befindet sich in der Schutzabdeckung 117 eine entsprechende Aussparung, so dass der Schließriegel 104 sich in seitlicher Richtung frei bewegen kann und aus der Schutzabdeckung 117 herausragen kann. Die Strahlenschutzanordnung 100 befindet sich in Figur 3 und 4 im Unterschied zu Figur 1 und 2 in verriegeltem Zustand. Dabei ragt der Schließriegel 104 über den Träger 116 in Richtung Trägerschiene 200 bzw. Behandlungstisch (beides nicht dargestellt) über den Träger 116 hervor. Ist die Strahlenschutzanordnung 100 in diesem Zustand auf eine Trägerschiene 200 aufgesteckt, so kann in dieser zweiten, geschlossenen Stellung des Haltegriffs 103 der Schließriegel 104 so angeordnet werden, dass er sich teilweise oder ganz über die Unterseite der Trägerschiene 200 erstreckt. Die Strahlenschutzanordnung ist damit verriegelt und wird an einem unbeabsichtigten Herunterfallen gehindert. Die zweite, geschlossene Stellung des Haltegriffs 103 ist nach unten gerichtet, im Winkel von etwa -90° im Bezug zur Längsrichtung des Längsprofils 105. Dabei befindet sich der Haltegriff 103 in einer entsprechenden Aussparung der Halterung 115, so dass der Haltegriff 103 mit einer Seite der Halterung 115 bündig abschließt. Erfindungsgemäß ist es jedoch auch möglich, dass in einer geschlossenen Position der Haltegriff 103 noch weiter in die Halterung eingeschwenkt ist, so dass sich der Haltegriff 103 in einer dort entsprechenden Aussparung befindet und bündig mit der Unterseite des Halters abschließt.

In dem in Figur 4 gezeigten Ausführungsbeispiel weist die Verbindungsstange 111 einen Sicherungsring 111b auf, der in einem kleinen Abstand vom Ende der Verbindungsstange 111 derart angeordnet ist, dass durch den Sicherungsring 111b ein Anschlagspunkt der Verbindungsstange 111 an dem Verbindungsstück 115' im unverriegelten Zustand des Haltegriffs 103 gebildet wird. Desweiteren weist die Verbindungsstange 111 an dem Ende, das mit dem Exzenter 115a in Kontakt steht, ein Endstück 111a auf. Das Endstück 111a der Verbindungsstange ist vorzugsweise kugelkopfförmig ausgebildet und besteht aus einem vorzugsweise selbstschmierenden und/oder gleitenden Material. Ein geeignetes Material kann beispielsweise Messing oder Teflon (PTFE) sein.

Figur 5 zeigt ein Set von zwei Strahlenschutzanordnungen 100 im verriegelten Zustand an der Trägerschiene 200 im Seitenbereich und im Kopfbereich eines Behandlungstisches gemäß einer zweiten Ausführungsform der vorliegenden Erfindung. In dieser zweiten Ausführungsform befindet sich jeweils eine Strahlenschutzanordnung 100 mit zwei Haltegriffen 103, 103' an der kurzen Seite und der langen Seite des Längsprofils. In diesem Fall steht der Haltegriff 103 als auch der erste Haltegriff 103' in Verbindung mit einem Schließmechanismus gemäß der ersten bevorzugten Ausführungsform, wie in den Figuren 1 bis 4 vorhergehend beschrieben. Somit existieren bei jeder Strahlenschutzanordnung 100 mindestens ein Schließriegel, zwei Haltegriffe und zwei Halterungen.

Erfindungsgemäß können Trägerschienen 200 an allen Längs- und Schmalseiten eines Behandlungstisches angebracht werden, so auch beispielsweise im Fußbereich des Behandlungstisches. Ferner kann die Trägerschiene 200 an einer oder mehreren Seiten des Behandlungstisches unterbrochen sein oder es können mehrere Trägerschienen 200 an einer oder mehreren gleichen Seiten des Behandlungstisches angebracht sein. Erfindungsgemäß ist es möglich mehrere Strahlenschutzanordnungen 100 nebeneinander an einer oder mehreren gleichen Seiten des Behandlungstisches anzubringen, beispielsweise zwei Strahlenschutzanordnungen 100 nebeneinander an einer Längsseite des Behandlungstisches.

Zusätzlich können wie gezeigt, erste Aufsetzlamellen 118a und zweite Aufsetzlamellen 118b (eine gezeigt) zum Schutz des Oberkörpers des Fachpersonals vor Strahlung angebracht werden. Die ersten Aufsetzlamellen 118a sind an der Längsseite des Behandlungstisches angeordnet, weisen einen Knick hin zum Behandlungstisch auf und sind zweiteilig ausgestaltet. Die zweite Aufsetzlamelle 118b ist breiter und weist eine gerade Grundform auf. Sie ist an der Schmalseite des Behandlungstisches angeordnet. Die ersten Aufsetzlamellen 118a und die zweiten Aufsetzlamellen 118b sind vorzugsweise derart angebracht und weisen vorzugsweise solche Abmessungen auf, dass wenn die unteren Endabschnitte der Aufsetzlamellen 118a,b überlappend mit den oberen Endabschnitten der Lamellen 101 zueinander ausgerichtet sind, die Endabschnitte der Lamellen 101 abgedeckt sind. Die Aufsetzlamellen 118a, 118b weisen an ihrer Unterseite eine oder mehrere Zapfen auf (nicht gezeigt), die geeignet sind in Bohrungen 114 an der Oberseite des Halters gesteckt zu werden. Die Aufsetzlamellen 118a und 118b können auf andere Weise auf dem Halter bzw. dem Längsprofil 105 befestigt werden, vorzugsweise mittels U-förmigen Halteeinrichtungen, die nachstehend näher erläutert werden.

Die Bohrungen 114 haben beispielsweise alternierende Abstände, können aber gemäß einer anderen Ausführungsform äquidistante Abstände haben. Die Bohrungen 114 bilden somit eine Lochreihe auf der Strahlenschutzanordnung 100 bzw. auf den nebeneinander angeordneten Strahlenschutzanordnungen 100, so dass die Aufsetzlamellen 118a, 118b variabel entlang der Lochreihe angeordnet werden können. So ist es beispielsweise möglich während einer Operation eine Aufsetzlamelle 118a, 118b derart zu verschieben, dass ein Spalt zwischen zwei Aufsetzlamellen 118a, 118b entsteht, durch den der Patient versorgt werden kann. Dieser Spalt kann nach der Versorgung wieder geschlossen werden, indem die verschobene Aufsetzlamelle 118a, 118b wieder an ihre ursprüngliche Position zurückgesetzt wird.

Erfindungsgemäß ist es auch möglich die Lochreihe der Bohrungen 114 durch andere Mittel zum Anordnen und Festhalten der Aufsetzlamellen 118a, 118b zu ersetzen. So kann jede Aufsetzlamelle 118a, 118b eine Befestigungseinrichtung aufweisen, die formschlüssig und / oder kraftschlüssig an dem Halter befestigbar ist. Beispielsweise weist die Befestigungseinrichtung ein U-Profil auf, das auf das Längsprofil 105 aufsetzbar ist. Vorzugsweise ist das U-Profil wie eine Klammer ausgebildet, und weist Rastmittel auf, die beispielsweise mit einer Einrastrille an der Außenseite des Längsprofils 105 lösbar in Eingriff bringbar ist. Dabei ist es vorteilhaft, dass mindestens die Einrastrille oder die Ausstülpung bzw. Aussparung aus einem elastischen Material, beispielweise einem Kunststoff besteht.

In die V-förmigen Aussparungen 110 der Strahlenschutzanordnungen 100 ragen die Bolzen 201 der Trägerschiene 200 herein, so dass eine etwaige Längsverschiebung der Strahlenschutzanordnung 100 an der Trägerschiene 200 verhindert werden kann.

In einer weiteren Ausführungsform weist die Strahlenschutzanordnung 100 zwei schwenkbare Haltegriffe 103, 103' auf, wobei jedoch nur der Haltegriff 103 mit einem Schließmechanismus in Wirkverbindung steht. Der andere Haltegriff 103' ist lediglich in der Halterung 115' schwenkbar gelagert und kann dementsprechend in die erste und zweite Position eingeschwenkt werden, ohne jedoch einen Schließmechanismus dadurch zu betätigen.

Durch die Verwendung einer variablen Befestigung der Strahlenschutzanordnung 100 an der Trägerschiene 200 durch die vorstehend beschriebene Verriegelung und der Verwendung der variablen Anordnung von Aufsetzlamellen 118a, 118b auf dem Halter der Strahlenschutzanordnung wird ein optimaler Schutz des Bedien- bzw. Fachpersonals vor Strahlung gewährleistet bei gleichzeitiger Anpassung des Strahlenschutzes an die Bedürfnisse des Bedien- bzw. Fachpersonals bzw. Anpassung an die spezifischen Gegebenheiten der jeweiligen Behandlung des Patienten.

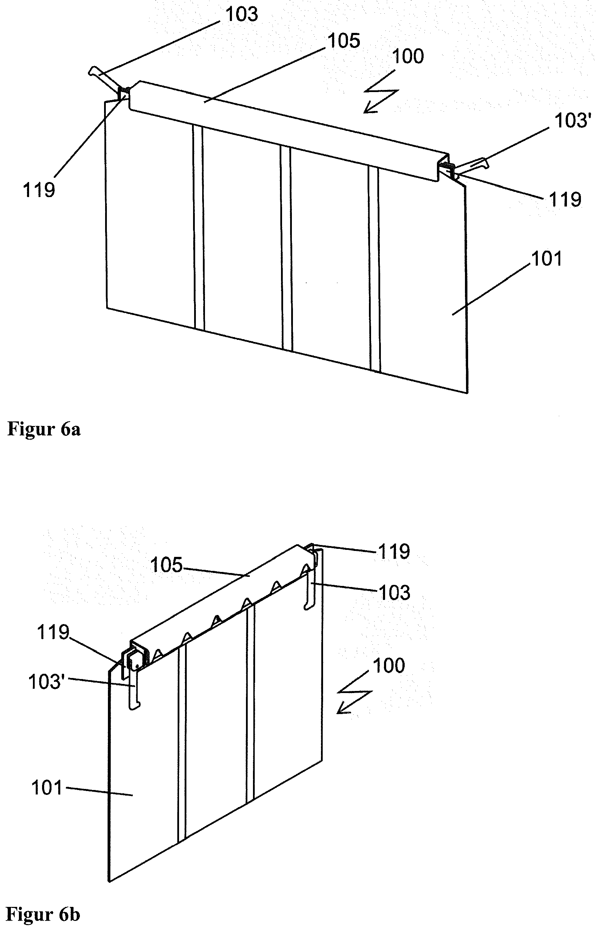

Figur 6a und 6b zeigen perspektivische Ansichten einer Strahlenschutzanordnung gemäß einer dritten bevorzugten Ausführungsform der vorliegenden Erfindung. Die Strahlenschutzanordnung 100 weist in dem gezeigten Beispiel vier Lamellen 101 auf, die jeweils an einer entsprechenden Befestigungsvorrichtung angebracht sind. Die Strahlenschutzanordnung 100 weist ferner einen Halter auf, wobei der Halter wiederum ein Längsprofil 105 aufweist. An dem Längsprofil 105 ist an der kurzen Seite seitlich auf beiden Seiten ein Haltegriff 103 bzw. 103' schwenkbar gelagert. Ferner weist die Strahlenschutzanordnung 100 eine Querlamelle 119 auf, die parallel zur langen Seite des Längsprofils 105 an dem Träger 116 (nicht gezeigt) angebracht ist. In dem Ausführungsbeispiel weisen die Lamellen 101 im Bereich des oberen Endes eine Dreieckform auf, die dreieckig in Richtung der Befestigungsvorrichtung zuläuft. Die Querlamelle 119 ist dabei derart angebracht und ausgestaltet, dass sie den oberen Teil der Lamellen 101 in Richtung des Behandlungstisches überlappt. Vorzugsweise deckt die Querlamelle 119 die Zone ab, in der der obere Teil der Lamellen 101 mit der Befestigungsvorrichtung verbunden ist. Das Längsprofil 105 weist in dem in den Figuren 6a und 6b gezeigten Beispiel keine Bohrungen 114 auf.

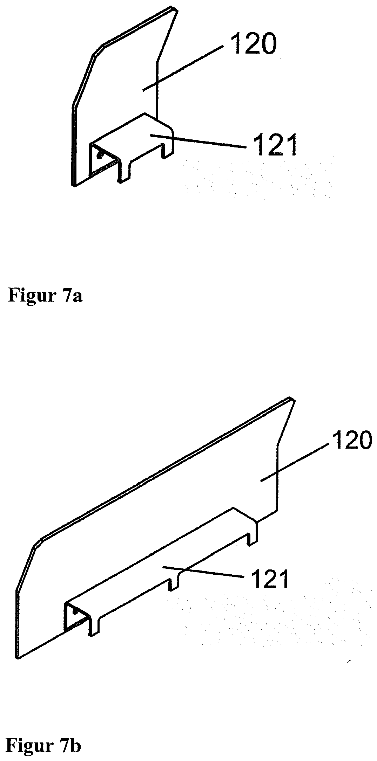

Figur 7a zeigt eine Aufsetzlamelle 120 gemäß einer weiteren bevorzugten Ausführungsform der vorliegenden Erfindung. An der Aufsetzlamelle 120 ist eine im Profil U-förmige Halteeinrichtung 121 angebracht, die vorzugsweise aus einem Kunststoff besteht. Durch diese U-förmige Halteeinrichtung kann die Aufsetzlamelle 120 an das Längsprofil 105 der Strahlenschutzanordnung 100 formschlüssig und/oder kraftschlüssig aufgesetzt werden. In dem vorliegenden Beispiel ist in der im Profil U-förmigen Halteeinrichtung an der zum Behandlungstisch zeigenden Seite eine Aussparung ausgeschnitten, so dass sich an dieser Seite nur noch zwei Stege befinden. Diese beiden Stege umfassen die zum Behandlungstisch zeigende Seite des Längsprofils 105, so dass eine stabile Halteeinrichtung gewährleistet ist.

Die Aufsetzlamelle 120 kann in Längsrichtung auf dem Längsprofil gleitend verschoben werden.

Figur 7b zeigt eine Aufsetzlamelle 120 gemäß einer weiteren bevorzugten Ausführungsform der vorliegenden Erfindung. Im Vergleich zur Aufsetzlamelle in Figur 7a ist die Aufsetzlamelle in dem in Figur 7b gezeigten Beispiel in Längsrichtung breiter, beispielhaft um einen Faktor 3. Dementsprechend ist auch die Halteeinrichtung 121 in dem in Figur 7b gezeigten Beispiel in Längsrichtung länger. Die Halteeinrichtung 121 weist an der in Richtung Behandlungstisch weisenden Seite zwei Aussparungen aus, so dass drei Stege ausgebildet sind. Diese drei Stege klammern die in Richtung Behandlungstisch zeigende Seite des Längsprofils 105, so dass ein sicherer Sitz der Aufsetzlamelle auf das Längsprofil 105 gewährleistet werden kann. Gemäß der vorliegenden Erfindung kann die Halteeinrichtung 121 auch nur einen oder auch mehr als drei Stege aufweisen. So kann die Anzahl und/oder die Breite der Stege je nach gewünschter Klemmkraft der Halteeinrichtung 121 gewählt und/oder variiert werden.

Auch in dem in Figur 7b gezeigten Ausführungsbeispiel, kann die Aufsetzlamelle in Längsrichtung auf dem Längsprofil 105 der Strahlenschutzanordnung 100 gleitend verschoben werden.

Obwohl die Erfindung mittels der Figuren und der zugehörigen Beschreibung dargestellt und detailliert beschrieben ist, sind diese Darstellung und diese detaillierte Beschreibung illustrativ und beispielhaft zu verstehen und nicht als die Erfindung einschränkend. Es versteht sich, dass Fachleute Änderungen und Abwandlungen machen können, ohne den Umfang und den Geist der folgenden Ansprüche zu verlassen. Insbesondere umfasst die Erfindung ebenfalls Ausführungsformen mit jeglicher Kombination von Merkmalen, die vorstehend oder nachfolgend zu verschiedenen Ausführungsformen genannt oder gezeigt sind.

Die Erfindung umfasst ebenfalls einzelne Merkmale in den Figuren auch wenn sie dort im Zusammenhang mit anderen Merkmalen gezeigt sind und/oder vorstehend oder nachfolgend nicht genannt sind. Auch können die in den Figuren und der Beschreibung beschriebenen Alternativen von Ausführungsformen und einzelne Alternativen deren Merkmale vom Erfindungsgegenstand beziehungsweise von den offenbarten Gegenständen ausgeschlossen sein. Die Offenbarung umfasst Ausführungsformen, die ausschließlich die in den Ansprüchen beziehungsweise in den Ausführungsbeispielen beschriebenen Merkmale umfasst sowie auch solche, die zusätzliche andere Merkmale umfassen.

## Patentansprüche

1. Strahlenschutzanordnung (100) insbesondere zur Anbringung an mindestens einer Trägerschiene (200), die vorzugsweise an einer oder mehreren Seiten eines Behandlungstisches angebracht ist, mit
einer Befestigungsvorrichtung (102) an der mindestens eine Lamelle (101), die ein Material zum Schutz vor Strahlung, vorzugsweise Röntgenstrahlung aufweist, vorzugsweise schwenkbar befestigt ist, wobei die Strahlenschutzanordnung (100) einen Halter, der auf die Trägerschiene (200) aufsetzbar ist, aufweist, wobei der Halter aufweist:
mindestens einen schwenkbar gelagerten Haltegriff (103), wobei der Haltegriff (103) ein freies Ende und ein Ende, an dem der Haltegriff (103) schwenkbar gelagert ist, aufweist,
einen Schließmechanismus geeignet zum Verriegeln des Halters an der Trägerschiene (200), wobei der Schließmechanismus durch eine Schwenkbewegung des mindestens einen an dem Halter schwenkbar gelagerten Haltegriffs (103) betätigbar ist,
ein Längsprofil (105), wobei das Längsprofil (105) eine kurze und eine lange Seite aufweist, der Haltegriff (103) an der kurzen Seite des Längsprofils (105) angebracht ist und wobei der Haltegriff (103) geeignet ist, parallel zur langen Seite des Längsprofils (105) des Halters hin und her geschwenkt zu werden.

2. Strahlenschutzanordnung (100) nach Anspruch 1, wobei der mindestens eine Haltegriff (103) in direkter Wirkverbindung mit dem Schließmechanismus steht.

3. Strahlenschutzanordnung nach Anspruch 1 oder 2, wobei der Schließmechanismus mindestens einen Schließriegel (104) aufweist, der geeignet ist den Halter an der Trägerschiene (200) zu verriegeln.

4. Strahlenschutzanordnung nach Anspruch 3, wobei der Haltegriff (103) geeignet ist in eine erste Stellung geschwenkt zu werden und wobei in der ersten Stellung der Halter an der Trägerschiene (200) durch den Schließriegel (104) nicht verriegelt ist.

5. Strahlenschutzanordnung nach Anspruch 3 oder 4, wobei der mindestens eine Haltegriff (103) geeignet ist in eine zweite Stellung geschwenkt zu werden und wobei in der zweiten Stellung der mindestens eine Schließriegel (104) den Halter an der Trägerschiene (200) verriegelt.

6. Strahlenschutzanordnung (100) nach einem der Ansprüche 3 bis 5, wobei der Halter eine Aufnahme (107) aufweist, wobei das Längsprofil (105) eine Auflagefläche (106) aufweist und, wenn die Strahlenschutzanordnung (100) auf die Trägerschiene (200) aufgesteckt ist, die Auflagefläche des Längsprofils (105) auf der Oberseite der Trägerschiene (200) teilweise aufliegt und wobei vorzugsweise der mindestens eine Schließriegel (104) sich in der Aufnahme (107) befindet, die an der Auflagefläche (106) des Längsprofils (105) derart angeordnet ist, dass sich der mindestens eine Schließriegel (104) in einem bestimmten Abstand A zur Auflagefläche (106) befindet.

7. Strahlenschutzanordnung (100) nach Anspruch 6, wobei der Abstand A derart ausgestaltet ist, dass wenn die Strahlenschutzanordnung (100) auf die Trägerschiene (200) aufgesteckt ist, der Schließriegel im verriegelten Zustand sich teilweise entlang der Unterseite der Trägerschiene erstreckt.

8. Strahlenschutzanordnung (100) nach Anspruch 6 oder 7, wobei der Abstand A derart ausgestaltet ist, dass wenn die Strahlenschutzanordnung (100) auf die Trägerschiene (200) aufgesteckt ist, der Schließriegel (104) im verriegelten Zustand an die Unterseite der Trägerschiene (200) gedrückt ist, vorzugsweise in einem Winkel von 45° zur Normalen der Unterseite der Trägerschiene (200).

9. Strahlenschutzanordnung (100) nach einem der Ansprüche 4 bis 8, wobei der mindestens eine Haltegriff (103) in der ersten Stellung in einer Position in einem Winkel von 10° bis 70°, vorzugsweise 30°, zur Längsrichtung des Längsprofils (200) des Halters bezogen auf die Oberseite des Längsprofils (200) angeordnet ist.

10. Strahlenschutzanordnung (100) nach einem der Ansprüche 5 bis 9, wobei der mindestens eine Haltegriff (103) in der zweiten Stellung in einer Position in einem Winkel von etwa -90° zur Längsrichtung des Längsprofils (200) des Halters bezogen auf die Oberseite des Längsprofils (200) angeordnet ist.

11. Strahlenschutzanordnung (100) nach einem der Ansprüche 1 bis 10, wobei der mindestens eine Haltegriff (103) an der kurzen Seite des Längsprofils (200) angebracht wird.

12. Strahlenschutzanordnung (100) nach einem der Ansprüche 1 bis 11, wobei der Halter eine Lagerung aufweist, in der der Haltegriff (103) schwenkbar gelagert ist.

13. Strahlenschutzanordnung (100) nach einem der Ansprüche 5 bis 12, wobei der Haltegriff (103) mindestens eine Rastaussparung (108) aufweist, die geeignet ist den Haltegriff (103) in der ersten und / oder der zweiten Stellung bis zu einer bestimmten Schwenkkraft zu halten.

14. Strahlenschutzanordnung (100) nach einem der Ansprüche 1 bis 13, wobei der Haltegriff (103) eine Abrutschsicherung (109) aufweist, die geeignet ist ein Abrutschen einer Hand von dem Haltegriff (103) in der zweiten Stellung zu verhindern, wobei die Abrutschsicherung (109) vorzugsweise als Vorsprung an dem freien Ende des Haltegriffs (103) ausgebildet ist.

15. Strahlenschutzanordnung (100) insbesondere zur Anbringung an mindestens einer Trägerschiene (200), die vorzugsweise an einer oder mehreren Seiten eines Behandlungstisches angebracht ist, mit einem Halter, der auf die Trägerschiene (200) aufsetzbar ist und mindestens einer Aufsetzlamelle (118a,b, 120), die an unterschiedlichen Positionen auf dem Halter anbringbar ist, wobei die Strahlenschutzanordnung (100) mit einer Strahlenschutzanordnung (100) nach einem der Ansprüche 1 bis 14 kombiniert ist.

16. Strahlenschutzanordnung (100) nach Anspruch 15, wobei der Halter mindestens eine Bohrung (114) und die mindestens eine Aufsetzlamelle (118a,b) mindestens einen Zapfen aufweist und wobei der mindestens eine Zapfen in die mindestens eine Bohrung (114) einsteckbar ist.

17. Strahlenschutzanordnung (100) nach Anspruch 15, wobei die mindestens eine Aufsetzlamelle (120) mindestens eine U-förmige Halteeinrichtung (121), vorzugsweise aus einem Kunststoff, aufweist und wobei die mindestens eine U-förmige Halteeinrichtung (121) auf den Halter aufsetzbar ist.

18. Strahlenschutzanordnung (100) nach einem der Ansprüche 1 bis 17 mit mindestens einer Querlamelle (119), die an der Befestigungsvorrichtung (102) angebracht ist, wobei die Querlamelle (119) vorzugsweise derart ausgestaltet und/oder angeordnet ist, dass die Querlamelle mit einem oberen Teil der Lamelle (101) überlappt.

## Claims

1. A radiation protection arrangement (100), in particular for attachment to at least one support rail (200), which is preferably attached to one or more sides of a treatment table, comprising
a fastening device (102) on the at least one lamella (101) which comprises a material for protection from radiation, preferably X-ray radiation, and which is preferably pivotably mounted, wherein the radiation protection arrangement (100) comprises a holder which can be attached to the support rail (200), wherein the holder comprises:
at least one pivotably mounted handle (103), the handle (103) having a free end and an end on which the handle (103) is pivotably mounted,
a locking mechanism suitable for locking the holder on the support rail (200), the locking mechanism being operable by a pivoting motion of the at least one handle (103) which is pivotably mounted on the holder,
a longitudinal profile (105), wherein the longitudinal profile (105) has a short and a long side, the handle (103) is attached to the short side of the longitudinal profile (105) and wherein the handle (103) is suitable for being pivoted to and from in parallel to the long side of the longitudinal profile (105) of the holder.

2. Radiation protection arrangement (100) according to claim 1, wherein the at least one handle (103) is in direct operative connection with the locking mechanism.

3. Radiation protection arrangement according to claim 1 or 2, wherein the locking mechanism comprises at least one locking bolt (104) suitable for locking the holder on the support rail (200).

4. Radiation protection arrangement according to claim 3, wherein the handle (103) is suitable for being pivoted into a first position and wherein, in the first position, the holder on the support rail (200) is not locked by the locking bolt (104).

5. Radiation protection arrangement according to claim 3 or 4, wherein the at least one handle (103) is suitable for being pivoted into a second position and wherein in the second position the at least one locking bolt (104) locks the holder on the support rail (200).

6. Radiation protection arrangement (100) according to any one of claim 3 to 5, wherein the holder comprises a receptacle (107), wherein the longitudinal profile (105) has a contact face (106) and, when the radiation protection arrangement (100) is attached to the support rail (200), the contact face of the longitudinal profile (105) partially rests on the upper side of the support rail (200), and wherein preferably the at least one locking bolt (104) is situated in the receptacle (107) which is arranged at the contact face (106) of the longitudinal profile (105) such that the at least one locking bolt (104) is situated at a particular distance A relative to the contact face (106).

7. Radiation protection arrangement (100) according to claim 6, wherein the distance A is arranged such that when the radiation protection arrangement (100) is attached to the support rail (200), the locking bolt in the locked state extends partially along the bottom of the support rail.

8. Radiation protection arrangement (100) according to claim 6 or 7, wherein the distance A is arranged such that when the radiation protection arrangement (100) is attached to the support rail (200), the locking device (104) in the locked state is pressed against the bottom of the support rail (200), preferably at an angle of 45° to the normal of the bottom of the support rail (200).

9. Radiation protection arrangement (100) according to any one of claims 4 to 8, wherein, in the first position, the at least one handle (103) is arranged in a position at an angle of from 10° to 70°, preferably 30°, to the longitudinal direction of the longitudinal profile (200) of the holder with regard to the upper side of the longitudinal profile (200).

10. Radiation protection arrangement (100) according to any one of claims 5 to 9, wherein, in the second position, the at least one handle (103) is arranged in a position at an angle of approximately -90° to the longitudinal direction of the longitudinal profile (200) of the holder with regard to the upper side of the longitudinal profile (200).

11. Radiation protection arrangement (100) according to any one of claims 1 to 10, wherein the at least one handle (103) is attached to the short side of the longitudinal profile (200).

12. Radiation protection arrangement (100) according to any one of claims 1 to 11, wherein the holder has a bearing in which the handle (103) is pivotably mounted.

13. Radiation protection arrangement (100) according to any one of claims 5 to 12, wherein the handle (103) has at least one latching recess (108) which is suitable for retaining the handle (103) in the first and/or the second position up to a specific pivot force.

14. Radiation protection arrangement (100) according to any one of claims 1 to 13, wherein the handle (103) comprises an anti-sliding device (109) which is suitable for preventing that a hand slides off from the handle (103) in the second position, the anti-sliding device (109) being preferably formed as projection at the free end of the handle (103).

15. Radiation protection arrangement (100), in particular for attaching to at least one support rail (200) which is preferably attached to one or more sides of a treatment table, comprising a holder which can be attached to the support rail (200) and at least one detachable lamella (118a,b, 120) which can be attached to the holder at different positions, wherein the radiation protection arrangement (100) can be combined with a radiation protection arrangement (100) according to any one of claims 1 to 14.

16. Radiation protection arrangement (100) according to claim 15, wherein the holder has at least one bore (114) and the at least one detachable lamella (118a,b) has at least one pin and wherein the at least one pin is insertable into the at least one bore (114).

17. Radiation protection arrangement (100) according to claim 15, wherein the at least one detachable lamella (120) has at least one U-shaped holding means (121), preferably made from a plastic, and wherein the at least one U-shaped holding means (121) is attachable to the holder.

18. Radiation protection arrangement (100) according to any one of claims 1 to 17 with at least one transverse lamella (119) attached to the fastening device (102), wherein the transverse lamella (119) is preferably designed and/or arranged such that the transverse lamella overlaps with an upper part of the lamella (101).

## Revendications

1. Ensemble de protection contre les radiations (100), notamment destiné à être placé sur au moins un rail porteur (200), qui est de préférence agencé sur un ou plusieurs côtés d'une table de traitement, l'ensemble comprenant
un dispositif de fixation (102) sur lequel est fixée, de préférence de manière pivotante, au moins une lamelle (101), qui comprend un matériau pour la protection contre les radiations, de préférence des rayons X, l'ensemble de protection contre les radiations (100) comportant un support qui peut être placé sur le rail porteur (200), ledit support comportant :
au moins une poignée de maintien (103) montée pivotante, la poignée de maintien (103) présentant une extrémité libre et une extrémité au niveau de laquelle la poignée de maintien (3) est montée pivotante,
un mécanisme de fermeture adapté à verrouiller le support sur le rail porteur (200), le mécanisme de fermeture pouvant être actionné par un mouvement de pivotement de ladite au moins une poignée de maintien (103) montée pivotante sur le support,
un profilé longitudinal (105), le profilé longitudinal (105) présentant un côté court et un côté long, la poignée de maintien (103) étant placée sur le côté court du profilé longitudinal (105), et la poignée de maintien (103) étant adaptée à pivoter en va et vient parallèlement au côté long du profilé longitudinal (105) du support.

2. Ensemble de protection contre les radiations (100) selon la revendication 1, dans lequel ladite au moins une poignée de maintien (103) est en interaction directe avec le mécanisme de fermeture.

3. Ensemble de protection contre les radiations selon la revendication 1 ou la revendication 2, dans lequel le mécanisme de fermeture présente au moins un verrou de fermeture (104), qui est adapté à verrouiller le support sur le rail porteur (200).

4. Ensemble de protection contre les radiations selon la revendication 3, dans lequel la poignée de maintien (103) est adaptée à être pivotée dans une première position, et dans lequel, dans la première position, le support n'est pas verrouillé sur le rail porteur (200) par le verrou de fermeture (104).

5. Ensemble de protection contre les radiations selon la revendication 3 ou la revendication 4, dans lequel ladite au moins une poignée de maintien (103) est adaptée à être pivotée dans une deuxième position, et dans lequel, dans la deuxième position, ledit au moins un verrou de fermeture (104) verrouille le support sur le rail porteur (200).

6. Ensemble de protection contre les radiations (100) selon l'une des revendications 3 à 5, dans lequel le support comporte un élément de réception (107), dans lequel le profilé longitudinal (105) présente une surface d'appui (106) et, lorsque l'ensemble de protection contre les radiations (100) est emmanché sur le rail porteur (200), la surface d'appui du profilé longitudinal (105) repose partiellement sur le côté supérieur du rail porteur (200), et dans lequel ledit au moins un verrou de fermeture (104) se trouve de préférence dans l'élément de réception (107), qui est agencé sur la surface d'appui (106) du profilé longitudinal (105) de manière telle, que ledit au moins un verrou de fermeture (104) se trouve à une distance A déterminée de la surface d'appui (106).

7. Ensemble de protection contre les radiations (100) selon la revendication 6, dans lequel la distance A est configurée de façon telle, que lorsque l'ensemble de protection contre les radiations (100) est emmanché sur le rail porteur (200), le verrou de fermeture, dans l'état verrouillé, s'étende partiellement le long du côté inférieur du rail porteur.

8. Ensemble de protection contre les radiations (100) selon la revendication 6 ou la revendication 7, dans lequel la distance A est configurée de façon telle, que lorsque l'ensemble de protection contre les radiations (100) est emmanché sur le rail porteur (200), le verrou de fermeture (104), dans l'état verrouillé, soit pressé contre le côté inférieur du rail porteur (200), de préférence sous un angle de 45° par rapport à la normale au côté inférieur du rail porteur (200).

9. Ensemble de protection contre les radiations (100) selon l'une des revendications 4 à 8, dans lequel ladite au moins une poignée de maintien (103), dans ladite première position, est agencée dans une position de manière à former un angle de 10° à 70°, de préférence de 30°, par rapport à la direction longitudinale du profilé longitudinal (200) du support, en se référant au côté supérieur du profilé longitudinal (200).

10. Ensemble de protection contre les radiations (100) selon l'une des revendications 5 à 9, dans lequel ladite au moins une poignée de maintien (103), dans ladite deuxième position, est agencée dans une position de manière à former un angle d'environ -90° par rapport à la direction longitudinale du profilé longitudinal (200) du support, en se référant au côté supérieur du profilé longitudinal (200).

11. Ensemble de protection contre les radiations (100) selon l'une des revendications 1 à 10, dans lequel ladite au moins une poignée de maintien (103) est agencée sur le côté court du profilé longitudinal (200).

12. Ensemble de protection contre les radiations (100) selon l'une des revendications 1 à 11, dans lequel le support comporte un système de palier où est montée pivotante la poignée de maintien (103).

13. Ensemble de protection contre les radiations (100) selon l'une des revendications 5 à 12, dans lequel la poignée de maintien (103) présente au moins un évidement d'encliquetage (108), qui est adapté à maintenir la poignée de maintien (103) dans ladite première et/ou deuxième position jusqu'à une force de pivotement déterminée.

14. Ensemble de protection contre les radiations (100) selon l'une des revendications 1 à 13, dans lequel la poignée de maintien (103) présente une sécurité de glissement (109), qui est adaptée à empêcher une main de glisser de la poignée de maintien (103) dans la deuxième position, la sécurité de glissement (109) étant de préférence réalisée sous forme de protubérance à l'extrémité libre de la poignée de maintien (103).

15. Ensemble de protection contre les radiations (100), notamment destiné à être placé sur au moins un rail porteur (200), qui est de préférence agencé sur un ou plusieurs côtés d'une table de traitement, et comprenant un support, qui peut être rapporté sur le rail porteur (200), et au moins une lamelle à rapporter (118a,b, 120), qui peut être placée dans différentes positions sur le support, l'ensemble de protection contre les radiations (100) étant combiné avec un ensemble de protection contre les radiations (100) selon l'une des revendications 1 à 14.

16. Ensemble de protection contre les radiations (100) selon la revendication 15, dans lequel le support présente au moins un alésage (114) et ladite au moins une lamelle à rapporter (118a,b) comporte au moins un tourillon, et dans lequel ledit au moins un tourillon peut être emmanché dans ledit au moins un alésage (114).

17. Ensemble de protection contre les radiations (100) selon la revendication 15, dans lequel ladite au moins une lamelle à rapporter (120) présente au moins un système de maintien (121) en forme de U, de préférence en une matière plastique, et dans lequel ledit au moins un système de maintien (121) en forme de U peut être rapporté sur le support.

18. Ensemble de protection contre les radiations (100) selon l'une des revendications 1 à 17, comprenant au moins une lamelle transversale (119), qui est placée sur le dispositif de fixation (102), ensemble dans lequel la lamelle transversale (119) est configurée et/ou agencée de préférence de manière telle, que la lamelle transversale chevauche une partie supérieure de la lamelle (101).
